# EUROPEAN PATENT APPLICATION

(11) **EP 3 666 320 A1**
(43) Date of publication of application: **17.06.2020**
(21) Application number: 18211438.9
(22) Date of filing: 10.12.2018
(51) Int. Cl.: A61M 25/00, A61B 17/3203

(54) **CATHETER FOR DELIVERING AN AGENT**

(71) Applicant: Brussels Medical Device Center ASBL, 1050 Bruxelles (BE); Université Libre de Bruxelles, 1050 Bruxelles (BE)
(72) Inventor: CAUCHE, Nicolas, 1160 Bruxelles (BE); DELATTRE, Cecilia, 1080 Bruxelles (BE); LINTERMANS, Alan, 1180 Bruxelles (BE); MONFILS, Jean, 1030 Bruxelles (BE); DEVIERE, Jacques, 1404 Bornival (BE)
(74) Representative: Pronovem

(57) **Abstract**

Catheter (10) for delivering an agent, comprising a catheter body (11) comprising a first lumen (112) extending from a proximal end (101) to a distal end (102) and defining a longitudinal axis (103) and a first outlet (113) adjacent to the distal end and in fluid communication with the first lumen (112). The catheter comprises fluid deflecting means (132) configured to deflect a flow of the agent discharged from the first outlet (113) in a direction having a backwards component relative to the flow in the first lumen, the fluid deflecting means comprising a fluid deflecting surface (137).

## Description

### Technical field

The present invention is related to catheters for delivering an agent, such as a drug or other therapeutic agent, into an internal cavity of a human or animal body. The agent can be supplied in powder, liquid or gel form or as an aerosol. The present invention is particularly related to catheters for delivering an agent in the gastrointestinal (GI) tract.

### Background art

Catheters of the above kind are known from WO 2011/112971 and include a catheter body containing a delivery lumen for transporting material. The catheter body is malleable at room temperature and holds its shape during insertion into the body lumen. A connector is located at the proximal end of the catheter body for connecting the device to a reservoir, and a tip is located at the distal end of the catheter body for insertion into a body lumen. The tip includes axial and/or radial exit holes for delivery of therapeutic material transported through the catheter body.

Gastrointestinal bleeding, in particular in the oesophagus and the stomach, can have dramatic consequences if not timely treated. Medical guidelines advocate a treatment within 12 hours after patient presentation. However, data has shown that a significant portion of the patients have a delay greater than 24 hours before undergoing endoscopy. The mortality rate of acute variceal bleeding in the upper GI tract is around 20% at six weeks. (Source: Early application of haemostatic powder added to standard management for oesophagogastric variceal bleeding: a randomised trial, Mostafa Ibrahim et al., Gut 2018;0:1-10; doi:10.1136/gutjnl-2017-314653)

A first aid measure to stop gastrointestinal bleeding is to supply a hemostatic agent to the bleeding site, mostly in powder form. It has been found that early (e.g. within two hours) application of haemostatic powder improves clinical and endoscopic haemostasis. The haemostatic agent can be delivered through a catheter, such as those described above. One difficulty is that such catheters require vision of the internal lumen to deliver the powder at the adequate site. To this end, the presence of an endoscopist is required. However, a patient may need to wait a long time for such trained personnel to be available, which may lead to increasing mortality probabilities. Yet another difficulty is that some sites in the GI tract, in particular in the stomach are difficult to reach by the spray holes of the catheter. These sites are located at the rear of the catheter and would require the catheter to be bent backwards. However, without vision as through an endoscope, this would result in a difficult and time consuming task.

### Summary of the invention

It is therefore an aim of the present invention to provide a catheter allowing to deliver an agent that overcomes the above problems. It is an aim to provide such catheters that allow to easily supply a desired agent even in endoluminal areas which are difficult to reach and/or without requiring vision anymore. It is therefore an aim of the present invention to provide catheters for delivering an agent inside a body lumen which would possibly not require a trained endoscopist. It is an aim to provide such catheters allowing a faster and more effective delivery of an agent in order to stop bleeding earlier and potentially give higher survival rates to the patients, in particular in case of gastrointestinal bleeding.

It is further an aim of aspects of the present invention to reduce a risk of clogging of the catheter by the agent that is delivered, e.g. due to the fact that the agent is exposed to bodily fluids or moisture at a too early stage.

According to the invention, there is therefore provided a catheter as set out in the appended claims. The catheter comprises a catheter body which comprises a first lumen extending from a proximal end to a distal end. The first lumen defines a longitudinal axis. The catheter further comprises a first outlet adjacent to the distal end and in fluid communication with the first lumen.

According to a first aspect, the catheter comprises fluid deflecting means configured to deflect a flow of a (medicinal or therapeutic) agent discharged from the first outlet in a backwards and possibly radial direction relative to the flow of the agent in the first lumen. The (mean) velocity of the flow deflected by the fluid deflecting means hence has a component along the longitudinal axis oriented backwards relative to the flow through the first lumen, the latter being directed towards the proximal end. In addition, the (mean) flow velocity can have a normal component relative to the longitudinal axis (i.e., a radial component). Advantageously, the fluid deflecting means comprises a fluid deflecting surface, which is advantageously arranged opposite (i.e., facing) the first outlet. Advantageously, the fluid deflecting surface is inclined with respect to the longitudinal axis at an angle between 95° and 180°, advantageously between 105° and 175° measured from a distal side of the longitudinal axis. The fluid deflecting surface can be mounted at a fixed orientation, or can be movable between different orientations relative to the longitudinal axis, e.g. assuming a deployed configuration with an orientation as indicated above and a non-deployed configuration, e.g. for reducing bulkiness.

Such a disposition of the fluid deflecting surface allows for deflecting the agent delivered through the catheter in a backwards and possibly radial direction, so that areas at the rear of the catheter can be reached by the agent easily and without need of bending or excessively manipulating the catheter body. It will be convenient to note that bending a catheter, such as those in the prior art, would increase its likeliness to jam and clog.

Advantageously, the first fluid deflecting means is formed of, or comprises a fluid channel or chamber extending between the first outlet and a second outlet arranged at a proximal side compared to the first outlet. The first outlet fluidly connects the first lumen to the fluid channel. The first outlet can be arranged in a side wall of the first lumen, or at a distal end of the first lumen. The fluid channel advantageously extends parallel to the first lumen (e.g., parallel to the longitudinal axis). Advantageously, the fluid channel can completely surround the first lumen, e.g. the first channel can be concentric with the first lumen. With such arrangement, a wall of the fluid channel opposite the first outlet acts as fluid deflecting surface. By way of example, the first fluid deflecting means can be configured to impart a 180° change of flow direction to the flow discharged from the first lumen.

Advantageously, at the second outlet, possibly arranged downstream of the first outlet, a further fluid deflecting step can be provided, hence, downstream of the (first) fluid deflecting surface. The second fluid deflecting step can be configured to impart a change of flow direction in an opposite sense compared to the directional change imparted by the first fluid deflecting means, e.g. by an angle between 5° and 85°, advantageously an angle between 15° and 65° in an opposite sense compared to the flow direction imparted by the first fluid deflecting means. As a result, a radial component in addition to the backwards component is imparted to the fluid discharged from the second outlet. A total change of flow direction at the second outlet compared to the direction of flow in the first lumen can be between 95° and 175°.

To provide the second fluid deflecting step, the fluid deflection means can comprise a protrusion extending radially of the catheter body. The protrusion is advantageously arranged opposite the second outlet. The protrusion comprises a fluid deflecting surface opposite the second outlet that acts as a fluid impingement surface and configured to impart the above indicated change in flow direction.

Such a second fluid deflecting means hence allows for obtaining a two-step fluid deflection, which makes the deflection at the fluid deflecting surface easier and more efficient. Moreover, it becomes possible to protect the flow of agent in the second fluid deflecting means from the external environment more effectively, leading to improved operational performance and less risk of clogging due to exposure to external factors.

According to a second aspect, which can be provided in conjunction with, or independent of the first aspect above, the catheter comprises a valve configured to close the first lumen from the external environment. The valve may close the first lumen at the distal side, such as at the first outlet, or further upstream. The valve is advantageously a pressure-sensitive valve, which opens upon reaching a threshold (differential) pressure level in the first lumen. To this end, the valve may comprise a resilient and/or flexible membrane. The valve may be configured to close again once the (differential) pressure in the first lumen falls below the threshold.

With such a valve system, it becomes possible to close the first lumen to avoid exposure of the agent within the first lumen to bodily fluids and moisture during introduction and manipulation of the catheter in the body. As a result, a risk of clogging of the lumen due to reaction of the (powdery) agent with the external fluids is greatly reduced or avoided.

A method of treating gastrointestinal bleeding is described herein as well.

### Brief description of the figures

Aspects of the invention will now be described in more detail with reference to the appended drawings, wherein same reference numerals illustrate same features and wherein:
Figure 1 represents an overview of parts of the catheter according to aspects of the present invention;
Figure 2 represents a side view of a delivery system for endoluminal delivery of a medicinal agent according to a first embodiment;
Figure 3 represents a cross sectional view of the delivery system of Fig. 2 along section line A-A;
Figure 4 represents a cross sectional view of an alternative delivery system according to the present invention;
Figure 5 represents a cross sectional view of the distal end of a catheter comprising two delivery systems connected to a same delivery lumen, for spraying an agent in backward and forward direction;
Figure 6 represents a side view of a delivery system for endoluminal delivery of a medicinal agent according to a second embodiment;
Figure 7 represents a cross sectional view of the delivery system of Fig. 6 along section line B-B;
Figure 8 represents a perspective view of a delivery system for endoluminal delivery of a medicinal agent according to a third embodiment;
Figure 9 represents a cross sectional view of the delivery system of Fig. 8 along section line C-C;
Figure 10 represents a perspective view of a delivery system for endoluminal delivery of a medicinal agent according to a fourth embodiment;
Figure 11 represents a cross sectional view of the delivery system of Fig. 10 along section line D-D;
Figure 12 represents a cross sectional view of a supply system according to aspects of the invention, for use with the delivery systems as described herein;
Figure 13 represents a view of the stomach with a catheter according to aspects of the present invention inserted therein.

### Description of embodiments

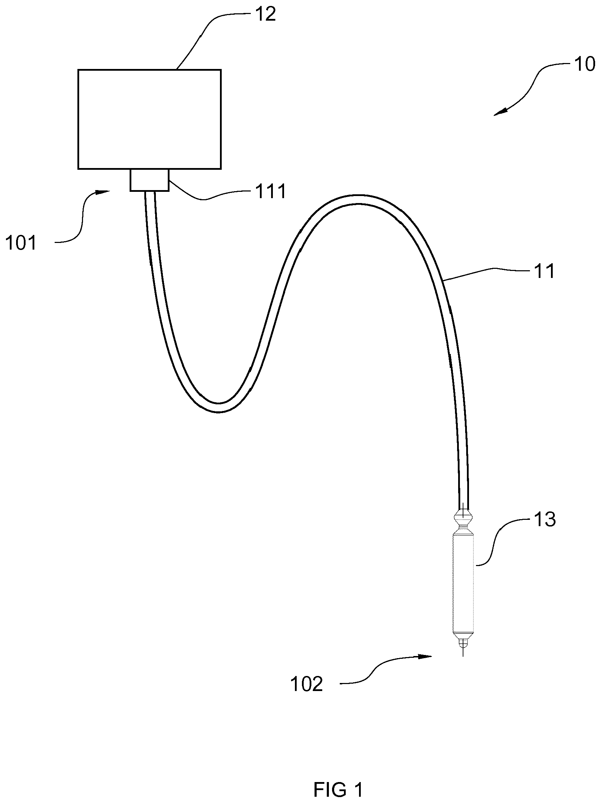

Referring to Fig. 1, a catheter 10 according to aspects of the present invention comprises a catheter body 11 that extends from a proximal end 101 to a distal end 102. The catheter body 11 is advantageously configured to be inserted in a lumen or cavity of a human or animal body through natural orifices, such as through the nasal cavity, into the GI tract. The catheter body 11 can comprise attachment means, such as a connector 111, at the proximal end 101 for attachment to a supply system 12 for supplying a medicinal or other, e.g. therapeutic, agent. At the distal end 102, the catheter body 11 is provided with a delivery system 13 for delivering the agent to the body. The catheter body 11 comprises a lumen extending from the proximal end 101 and in fluid connection with the supply system 12, to the delivery system 13.

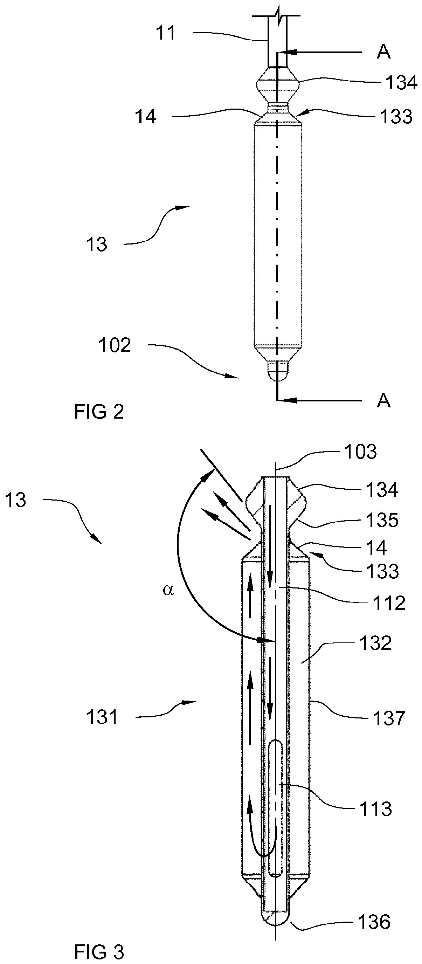

Referring to Figs. 2 and 3, a first embodiment of delivery system 13 has delivery lumen 112 extending through the catheter body 11 until the distal end 102. Lumen 112 defines a longitudinal axis 103 and is configured to transport the agent to a treatment site inside the body. Lumen 112 advantageously comprises a closed tip 136 at the distal end. The delivery system 13 comprises a fluid deflecting system 131 configured to deflect the forward flow of agent to a radial and backward flow. Fluid deflecting system 131 comprises a fluid channel or chamber 132 which is arranged at a circumference of lumen 112 and advantageously completely surrounds lumen 112 at the distal end. Lumen 112 comprises an outlet 113 for fluid communication with fluid chamber 132. Outlet 113 can be formed as one or more slots or holes provided in a wall of the lumen 112. Multiple of such slots or holes may be arranged at different angular positions around the wall of lumen 112. Alternatively, or in addition, it may be possible to open the tip 136 of lumen 112 towards fluid chamber 132.

The fluid chamber 132 extends from the tip 136 of lumen 112 to a second outlet 133 arranged at a proximal side compared to tip 136. The second outlet 133 is advantageously provided at an end of the fluid chamber 132 opposite the (first) outlet 113. Second outlet 133 opens to the body lumen or cavity to deliver the agent.

The delivery system 13 as shown in Figs. 2 and 3 advantageously has rotational symmetry about axis 103, e.g. symmetry with respect to a rotation over an angle of 180°, 120°, 90° or 45°. It may be convenient to make second outlet 133, as well as fluid impingement surface 135 as a circular aperture surrounding lumen 112 in order to deliver a medicinal agent in 360° directions around axis 103.

The agent, in gaseous, liquid or powder form is advantageously transported through lumen 112 with a carrying fluid, which may be liquid or gaseous, such as CO₂. An appropriate fluid/agent mixture is advantageously prepared in supply system 12. At the distal end, the fluid/agent mixture exits lumen 112 through outlet 113 and enters fluid chamber 132. An external wall 137 of fluid chamber 132 is advantageously arranged opposite outlet 113 to deflect fluid and agent discharged from the outlet 113 towards the outlet 133. The fluid/agent mixture then flows from outlet 113 to second outlet 133 from where it is discharged. The flow direction of the fluid/agent mixture hence makes a 180° turn in proximity of the outlet 113, since the fluid flow in the fluid chamber 132 will be in opposite direction with respect to the fluid flow in lumen 112, as indicated by the arrows in Fig. 3. At the second outlet 133, the fluid/agent mixture is discharged from fluid chamber 132 to advantageously reach treatment sites which are located behind the delivery system 13.

A fluid deflecting member 134 is advantageously arranged adjacent to second outlet 133. Fluid deflecting member 134 comprises a fluid impingement surface 135 facing the second outlet 133. Fluid that exits the fluid chamber 132 though outlet 133 impinges on surface 135 and is deflected, advantageously in a radial direction. Fluid impingement surface 135 therefore adds a further directional change to the fluid flowing through the fluid chamber 132, e.g. to increase the spray angle. Fluid impingement surface 135 is advantageously inclined relative to the longitudinal axis 103 at an angle α advantageously falling between 95° and 175°, advantageously between 100° and 170°, advantageously between 105° and 165°, advantageously between 110° and 160°, advantageously between 115° and 155° measured as from a distal (forward) side of the longitudinal axis 103. Doing so, a broad spray angle is advantageously obtained allowing for effectively treating areas of the GI tract that are located at the rear of the delivery system 13 and without having to bend or deflect the catheter body. Endoscopic vision during delivery of the agent is not required since it is ensured that the target areas located at the rear of the delivery system are treated. Furthermore, catheter manipulation is eased, reducing treatment time.

A valve 14 is advantageously arranged at the second outlet 133, or possibly at an upstream location in fluid chamber 132. Valve 14 is advantageously a pressure-sensitive valve arranged to open the outlet 133 when a predetermined pressure level is reached in fluid chamber 132. Alternatively, valve 14 can be a remotely actuated valve, e.g. connected to the proximal end, such as through an actuating cable, to open the valve 14 when desired. The valve 14 advantageously prevents bodily fluids and moisture to enter the catheter and the delivery system 13 in particular during insertion of the catheter in the body and during manipulation of the catheter until it reaches the desired treatment site. This is particularly advantageous when the agent that is to be administered reacts under influence of these external fluids, and particularly when the agent is in powder form, as there would be a risk of clogging the catheter if the bodily fluids or moisture would enter the catheter.

For a pressure-sensitive valve, the threshold pressure level for opening the valve can advantageously be set to a working pressure level of the supply system 12, e.g. between 1 and 8 bar such that the valve opens when the supply system is turned on. Valve 14 can e.g. be formed as a resilient membrane that covers the outlet and is pushed away from the outlet by a suitable differential pressure between the fluid chamber 132 and the treatment site.

As an alternative, a seal can be provided instead of valve 14 to close the second outlet 133. The seal is advantageously pressure-sensitive, in the sense that it opens the outlet once a predetermined pressure level is attained in the fluid chamber 132. A seal is typically for single use, as it may not able to close the outlet again at the end of treatment and/or when pressure in the fluid chamber 132 is reduced, e.g. the seal is frangible at a predetermined pressure level.

In a yet alternative case, the valve 14, or a seal can be provided at the outlet 113, instead of the second outlet 133.

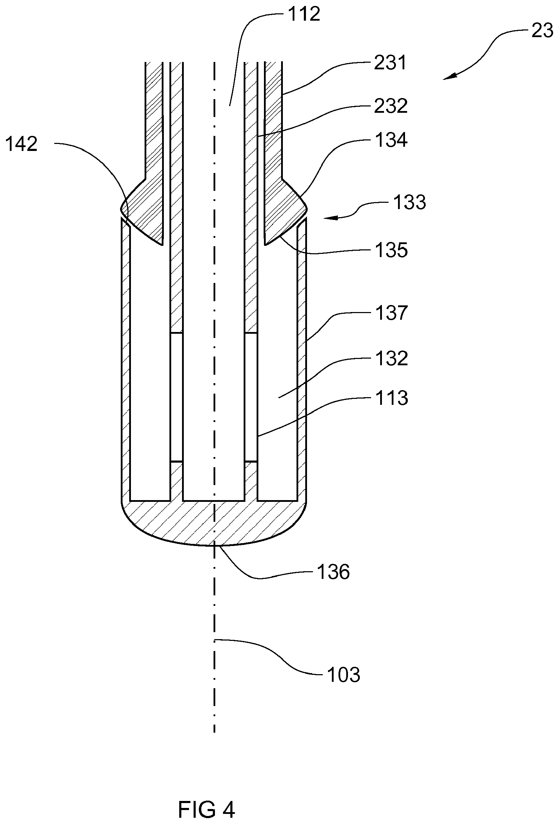

Yet alternatively, referring to Fig. 4, a valve can be created by forming the delivery system out of two parts which are moveable relative to each other in order to open and close the second outlet 133 and/or the (first) outlet 113. Delivery system 23 differs from delivery system 13 in that the fluid deflecting member 134 is fixedly attached to an outer sheath 231 that may extend until the proximal end 101. Lumen 112 is provided by an inner tube 232 which is slidingly arranged in the outer sheath 231. Fluid chamber 132 is fixedly attached to inner tube 232. By sliding the inner tube 232 relative to the outer sheath 231, e.g. until an outer wall 137 of fluid chamber 132 (i.e., edge 142 of the outlet 133) abuts against the fluid deflecting member 134, the second outlet 133 can be closed. Sliding in the opposite direction opens the second outlet 133 while the fluid deflecting member 134 acts as fluid deflector directing fluid discharged from the fluid chamber 132 along the impingement surface 135 in a radial direction. The fluid deflecting system in delivery system 23 is otherwise identical to fluid delivery system 13.

It can be contemplated to provide a third outlet at the tip 136 in the lumen 112 or fluid chamber 132, e.g. with a spray nozzle, opening in the distal direction. In such a case, the delivery system would allow both forward and backward spraying of the medicinal agent simultaneously.

The delivery system 13, in particular the lumen 112 and the fluid chamber 132 can be made of flexible materials, as this would ease guiding of the catheter through the body lumen or cavities. Alternatively, or in addition, the chamber 132 can be made of an inflatable material, configured to inflate due to the pressure exerted by the fluid discharged from outlet 113.

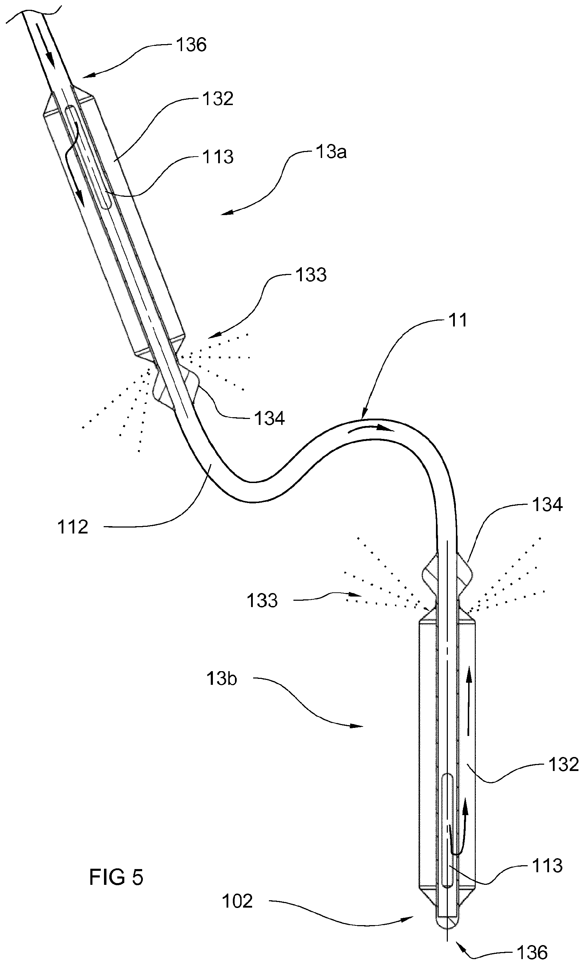

The catheter can comprise a plurality of delivery systems such as systems 13 or 23. These delivery systems may be positioned at axially distinct locations and can be coupled to a same delivery lumen 112, or to separate delivery lumens. The plurality of delivery systems may have different fluid deflection angles and/or be configured to spray in different directions. Referring to Fig. 5, the catheter can comprise two delivery systems 13a, 13b attached to the catheter body 11 at axially distinct locations, e.g. spaced apart from one another along the longitudinal axis of the catheter body 11. Both delivery systems 13a, 13b are fluidly coupled to the delivery lumen 112 to receive an agent/fluid mixture. A first delivery system 13a is arranged proximally of the second delivery system 13b. The second delivery system 13b can be identical to the ones described previously. The first delivery system 13a is connected to the lumen 112 in the inverse sense, e.g. with tip 136 arranged at the proximal side and the outlet 133 arranged at the distal side of the delivery system. The first delivery system 13a is open at the distal tip 136 where it is in fluid connection with the proximal part of lumen 112. At the distal side, lumen 112 crosses the first delivery system 13a and continues further to the second delivery system 13b. The flow of a fluid/agent mixture is indicated by the arrows, with the first delivery system 13a arranged to spray in forward direction (same direction as the flow in lumen 112) and the second delivery system 13b arranged to spray in backward direction compared to the flow direction in lumen 112, as indicated by the dotted lines in Fig. 5. Such a configuration advantageously allows for delivering the fluid/agent mixture to a larger area at once, covering the space between the first and second delivery systems.

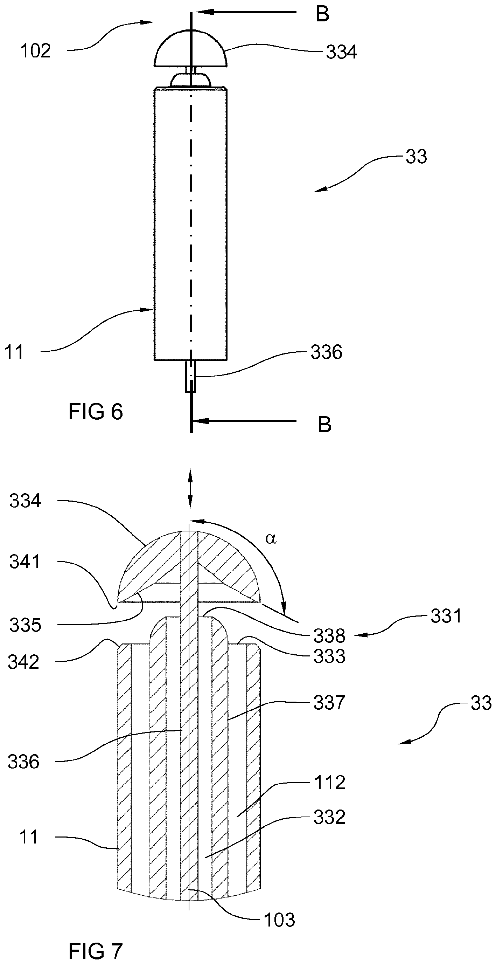

Referring to Figs. 6 and 7, an alternative delivery system 33 comprises a fluid deflecting system 331 that is somewhat different compared to the fluid deflecting system 131 of Figs. 2-4. Delivery system 331 comprises delivery lumen 112 formed in catheter body 11. An inner lumen 332 is arranged inside delivery lumen 112. Inner lumen 332 can be coaxial with delivery lumen 112, and delivery lumen 112 can completely surround inner lumen 332. Inner lumen 332 is formed by a fluid supply duct 337 which advantageously separates inner lumen 332 from delivery lumen 112. Alternatively, a plurality of delivery lumens can be arranged at a circumference of the fluid supply duct 337, e.g. at separate angular positions.

Delivery lumen 112 extends in the distal direction until outlet 333, from where a fluid/agent mixture is discharged. Fluid supply duct 337 advantageously extends in the distal direction until past the outlet 333 and discharges at fluid outlet 338. Fluid outlet 338 is advantageously arranged at a position along the longitudinal axis 103 which is more distal than the position of outlet 333. The fluid deflecting system further comprises a fluid deflecting member 334 which has an inner surface acting as a fluid impingement surface 335 which extends over (and covers) at least the fluid outlet 338, and preferably also over outlet 333. Fluid deflecting member 334 is advantageously formed as dome-shaped cap to cover the fluid outlet 338 (fluid supply duct 337) and preferably also outlet 333 (catheter body 11). The inner surface of the dome-shaped cap is in this case formed by the impingement surface 335. The impingement surface 335 hence faces at least the fluid outlet 338.

A push rod 336 is attached to the fluid deflecting member 334. Push rod 336 extends along the catheter body 11, e.g. within the inner lumen 332, until the proximal end 102, from where it can be actuated to move the fluid deflecting member 334 relative to the catheter body 11. Advantageously, a peripheral rim 341 of fluid deflecting member (cap) 334 conforms to a peripheral edge of the catheter body 11, in particular to a circumferential edge 342 of the outlet 333. Advantageously, a diameter of peripheral rim 341 of fluid deflecting member is substantially equal to an outer diameter of the catheter body. By so doing, the fluid deflecting member 334 can be pulled by push rod 336 to close the outlet 333 in order to prevent ingress of bodily fluids or moisture inside the delivery lumen 112, which may deteriorate the agent that is to be delivered. At the treatment site, the rod 336 can be pushed in distal direction relative to the catheter body 11 to open the outlet 333. As a result, the fluid deflecting member 334 and push rod 336 can act as a valve system.

In use, a fluid, which can be a gas or a liquid, is supplied through the inner lumen 332 and discharged from fluid outlet 338. As the impingement surface 335 is arranged opposite the fluid outlet 338, the fluid impinges on the surface 335 and is deflected radially outward, thereby passing the outlet 333, where it intersects with the agent, or fluid/agent mixture that is discharged through outlet 333. The fluid from outlet 338 and deflected by surface 335 will therefore entrain any fluid/agent mixture that is discharged from the outlet 333 into a radially outward direction. As a result, the agent is sprayed in a radial and backwards direction due to the orientation of the impingement surface 335.

Advantageously, only a fluid, such as a gas, and no agent is supplied through inner lumen. The agent is advantageously supplied only through the delivery lumen 112. One advantage of the inner lumen 332 is that it improves evacuation of a powdery agent that is discharged from the delivery lumen at outlet 333, avoiding the powder to heap up between the outlet 333 and the impingement surface. This is obtained with a delivery system 33 having very compact dimensions, e.g. dimensions which do not exceed those of the catheter body 11. It will therefore be convenient to note that the delivery system 33 advantageously has an outer cylindrical shape without protrusion, and with a substantially arcuate, spherical or at least atraumatic cap at the distal end.

Impingement surface 335 is appropriately inclined relative to the longitudinal axis 103 to obtain a deflection of the fluid which is radially outward and in a backward direction relative to the fluid flow in lumens 112 and 332. Advantageously impingement surface 335 is inclined at an angle α as indicated hereinabove and measured as from a distal (forward) side of the longitudinal axis 103.

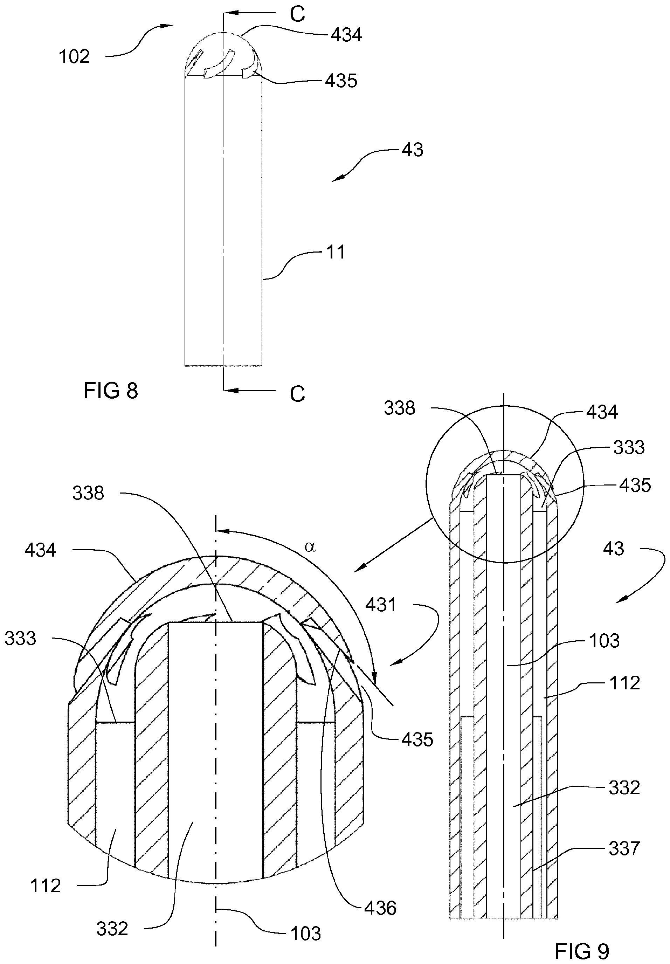

Referring to Figs. 8 and 9, yet another delivery system 43 according to aspects of the present invention differs from delivery system 33 in that the fluid deflecting system 431 comprises a fluid deflecting member 434 which is fixedly attached to the catheter body 11. Fluid deflecting member 434 forms a dome-shaped cap that covers the delivery lumen 112. Both delivery lumen 112 and inner lumen 332 are advantageously cylindrical shaped and concentric.

A plurality of exit holes 435 are arranged through the fluid deflecting member 434, advantageously at circumferential locations around the member. Exit holes 435 are in fluid communication with outlet 333 of delivery lumen 112 and fluid outlet 338 of inner lumen 332. Advantageously, the exit holes 435 are positioned at an intermediate position along the longitudinal axis 103 between outlet 333 and fluid outlet 338, and they may extend distally past the outlet 338. Advantageously, the exit holes 435 are arranged at a radial position substantially corresponding with the outlet 333, and may be arranged radially past the outlet 333.

The exit holes 435 comprise a fluid deflection surface 436 forming an inner wall of the exit holes. The fluid deflection surface is particularly formed by a distal portion of the inner wall of the exit holes. The fluid deflection surface 436 is inclined to deflect fluid discharged from fluid outlet 338 to the exit holes 435. By so doing, the flow of fluid crosses the fluid/agent mixture discharged from outlet 333 and entrains it towards the exit holes 435, from where the agent will be sprayed in a radially outward direction, and backwards, towards the proximal side. An angle of inclination α of the fluid deflection surface 436 is advantageously as indicated above in relation to the previously described embodiments.

Fluid discharged from fluid outlet 338 is deflected by the dome-shaped inner wall of the fluid deflecting member 434 towards the exit holes 435. The fluid will entrain the fluid/agent mixture supplied by delivery lumen 112 along the inner wall of member 434 towards the exit holes 435 from where it exits the delivery system 43 in a radially outward and backward direction compared to the proximal-distal direction. The fluid deflecting member 434 may further comprise one or more holes that are configured to spray a fluid/agent mixture in a forward direction. These holes may e.g. be interleaved with the exit holes 435.

The delivery system 43 does not comprise a valve, even though a pressure-sensitive valve closing the exit holes 435 may be contemplated. Entraining fluid, such as carbon dioxide gas can be continuously supplied through the inner lumen 332 during insertion and manipulation of the catheter inside the body in order to prevent ingress of bodily fluids of moisture through the exit holes 435. This delivery system 43 may be used along with a suction system, e.g. a suction pipe, in order to evacuate the supplied entraining fluid from the body and prevent damage to the body lumen or cavity by excessive insufflation.

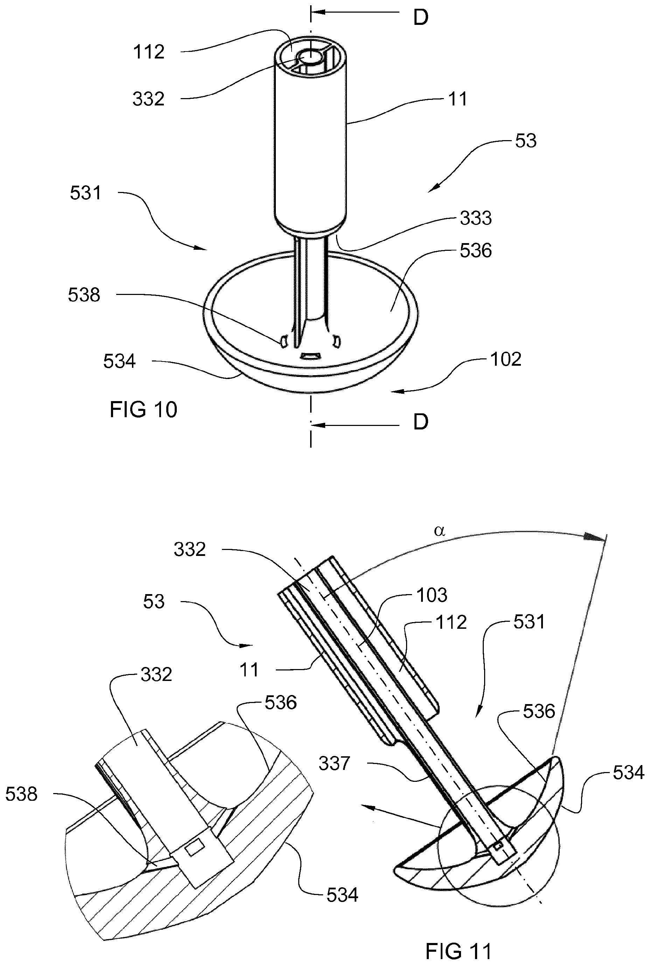

Referring to Figs. 10 and 11, an alternative delivery system 53 is shown that can be used in catheters 10 according to aspects of the present invention. Delivery system 53 differs from delivery system 43 in that the fluid deflection system 531 comprises an advantageously dome-shaped fluid deflecting member 534. An advantageously concave surface 536 of fluid deflecting member 534 is facing the outlet 333 of the delivery lumen 112 at a spaced apart distance. Advantageously, the concave surface 536 extends radially beyond the catheter body 11. That is, a diameter of an outer rim of the fluid deflecting member 534 is advantageously larger, e.g. at least 1.2 times larger, than an outer diameter of the catheter body 11, or than a diameter of the delivery lumen 112. The fluid supply lumen 332 is provided in an inner duct 337, just like in the embodiment of Figs. 6-7 and 8-9. The inner duct 337 extends in a distal direction past the outlet 333 of delivery lumen 112. Inner duct 337 may be coaxial and advantageously concentric with catheter body 11 which defines the delivery lumen 112.

The fluid deflecting member 534 is advantageously attached at a distal end of the inner duct 337. The fluid supply lumen 332 advantageously extends until the fluid deflecting member 534. The fluid supply lumen 332 advantageously comprises a plurality of outlet channels 538 extending substantially radially outwards relative to the longitudinal axis 103 to fluidly communicate with the concave surface 536. The outlet channels 538 may open flush with concave surface 536. Delivery system 53 may have rotational symmetry about the longitudinal axis 103, in particular a rotational symmetry with respect to a rotation by an angle of 180°, 120°, 90° or possibly 45°. With such a configuration, fluid discharged from outlet channels 538 and fluid discharged from outlet 333 will cross in a space between the outlet 333 and the surface 536.

In use, a fluid such as carbon dioxide gas is insufflated through the fluid supply lumen 332. The fluid exits the fluid supply lumen through the outlet channels 538 which convey it over the concave surface 536. Concave surface 536 advantageously directs the fluid in a radial and backwards (proximal) direction. The concave surface 536, in particular an outer edge portion thereof where fluid detaches from the concave surface, is advantageously inclined with respect to the longitudinal axis at an angle α as indicated hereinabove. Simultaneously, the agent is transported through the delivery lumen 112, e.g. with a carrying fluid as an agent/fluid mixture. The carrying fluid may be the same fluid as the fluid through fluid supply lumen 332. As the agent is discharged from outlet 333 in a direction towards the concave surface 536, it is advantageously entrained by the fluid exiting the outlet channels 538 and is deflected in a radial and backwards direction to reach treatment zones that are arranged at the rear of the delivery system 53. Furthermore, the outlet channels 538 advantageously remove any agent that may impinge on concave surface 536.

Since only fluid passes through the outlet channels 538, these can be made suitably small as there is a reduced risk of clogging. As with the previous embodiments, it can be contemplated to provide a valve (not shown) at the outlet 333 to prevent ingress of bodily fluids into the delivery lumen 112 during insertion and manipulation of the catheter inside the body. It will however be convenient to note that a valve may be omitted since the lumen 112 is advantageously straight at the distal end with the aperture of outlet 333 being in a plane substantially orthogonal to the longitudinal axis 103. Hence, in the delivery system 53, the deflection of the agent is made to occur between the outlet 333 and the concave surface 536, and hence outside of the system, such that risk of clogging of the agent, e.g. in case of powdery agents, is minimized.

The fluid deflecting member 534 can be made of a flexible or resilient material. It can be contemplated to make the member deployable, so as to reduce bulkiness during insertion through the body orifice.

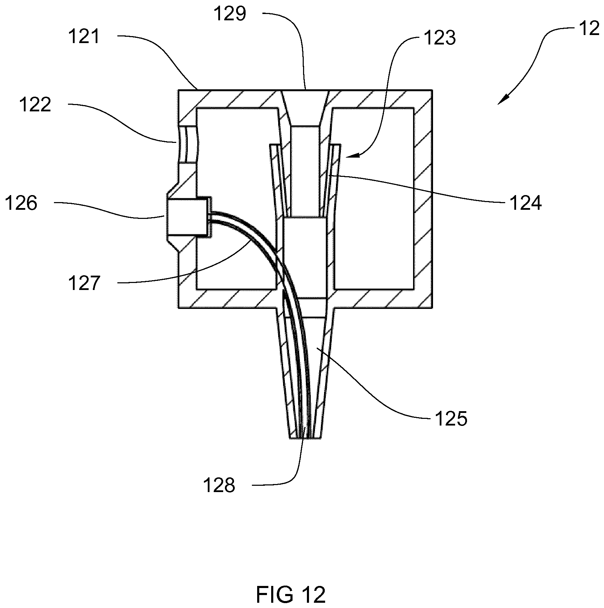

Any of the above delivery systems are advantageously used in combination with a supply system 12 as represented in Fig. 12. Supply system 12 is advantageously configured to prepare a mixture of agent and carrying fluid in suitable proportions. The fluid is advantageously a gas, such as carbon dioxide, but may alternatively be a liquid. The agent is advantageously a powder, but can alternatively be a liquid, in which case an aerosol can be obtained, or even a gas.

Supply system 12 as represented in Fig. 12 is advantageously suited for preparation of a mixture of gas and a powder agent. It comprises a fluid chamber 121 having a fluid (gas) input port 122. A Venturi mixing device 123 comprises a Venturi channel 124 arranged concentrically about an agent supply tube 129. The Venturi channel 124 is in fluid communication with the fluid chamber 121 at its inlet. Both the Venturi channel 124 and the agent supply tube 129 are in fluid communication with the output port 125 that delivers a mixture of gas and agent supplied from the input port 122 and the supply tube 129, respectively. The Venturi mixing device 123 is advantageously housed within the fluid chamber 121. The output port 125 is advantageously connected to the delivery lumen 112 of catheter body 11, e.g. through connector 111 (see Fig. 1).

One advantage of a Venturi mixing device is that it is a self-regulating system. If for whatever reason a clogging of the delivery lumen 112 occurs, the flow of gas through the Venturi is stopped, which will automatically stop the supply of agent. This prevents the delivery lumen to become completely filled with powder. As soon as the flow starts again, the agent is again delivered.

For the embodiments of Figs. 6-7, 8-9 and 10-11, the supply system 12 can additionally comprise a secondary fluid (gas) supply port 126, advantageously arranged in a wall of the fluid chamber 121. The secondary fluid supply port 126 is in fluid communication with a secondary fluid output port 128 through a duct 127 that advantageously is fluidly isolated from the fluid chamber 121. Fluid output port 128 is configured for being connected to the fluid supply lumen 332 through a suitable connection system as known in the art.

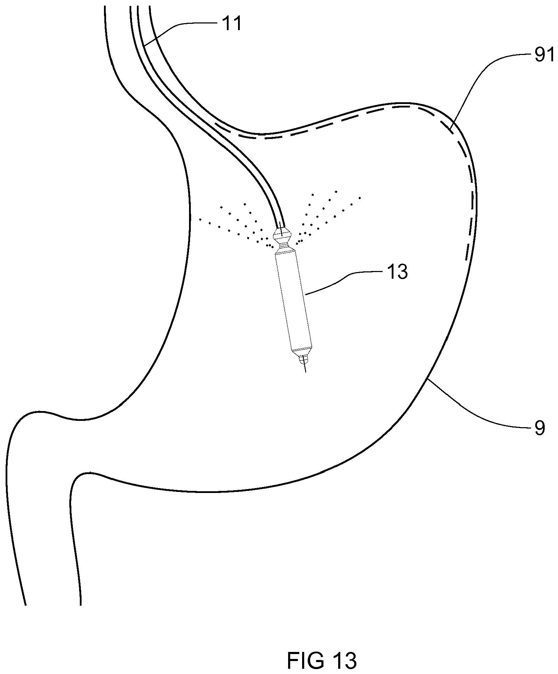

Referring to Fig. 13, devices as described herein are particularly advantageous for treatment of gastrointestinal bleeding through delivery of a haemostatic agent. To do so, the delivery system attached at the distal end of the catheter body is advantageously introduced in the body through the nasal cavity into the GI tract. The catheter body can be further introduced until it reaches the treatment site, e.g. the stomach 9. The position of the catheter body inside the body can be adjusted according to different methods. In one example, graduations are provided on the catheter body to indicate what length has been inserted allowing the practitioner to adjust the depth of the insertion according to the corpulence of the patient. For treatment in the stomach, the output of the (most distal) delivery system of the catheter should be inserted until about 55 cm from the dental arcade for tall persons and a minimum of about 45 cm for smaller persons. Alternatively, an inflatable balloon is attached to the catheter, which can be inflated when inside the stomach. The balloon is positioned relative to the delivery system such that, when the practitioner pulls on the catheter body until the balloon makes contact with the cardia, the catheter will be in the right position inside the upper gastrointestinal tract. The balloon can then be deflated. Yet alternatively, gas is supplied through the supply system 12, either through the delivery lumen 112, the fluid supply lumen 332, or both and without delivery of agent. The sound of the gas flow touching the gastric wall is heard using a stethoscope, and the operator may know when the delivery system arrives at the treatment site, e.g. the stomach. Other techniques to correctly position the catheter inside the GI tract without requiring vision can be contemplated.

At this point, the agent can be supplied to supply system 12, which may prepare a fluid/agent mixture that is delivered to the delivery system 13, from where it is delivered to the bleeding site 91. As shown in Fig. 13, the bleeding site 91 may well be located at the rear of the delivery system, e.g. the bleeding site may be surrounding the cardia, and catheters as described herein are particularly suitable to deliver such agents at such sites which are difficult to reach and without needing to bend the catheter, and without requiring visual feedback. Possibly, after or at the same time, the agent is sprayed distally, e.g. from another delivery system located proximally compared to the one delivery system, e.g. in order to deliver the agent in the oesophagus. Alternatively, a same delivery system can be used, which also allows to spray in forward (distal) direction. The latter step can be repeated several times or performed continuously while pulling the catheter body over several centimetres until at distance of 30 cm from the dental arcade. Finally, the catheter can be extracted in a non-traumatic manner. IT will be convenient to note that the catheter can also be used for veterinary applications.

The catheter body and/or delivery system are advantageously made of a biocompatible material, in particular a biocompatible polymer. Suitable examples of such polymers are polyurethane, polyvinyl chloride, polytetrafluoroethylene, polyamide, such as nylon, polyethylene terephthalate and polyethylene (in any of its forms e.g. as high, medium or low density). Alternatively, elastomeric biocompatible materials may be used as well, such as natural rubber, or thermoplastic elastomers.

The dimensions of the catheter body and of the delivery system are advantageously adapted to the dimensions of the natural orifice through which the catheter is introduced in the body. Advantageously, catheter body 11 has an external diameter of at most 6 mm, advantageously at most 5.5 mm, advantageously at most 5.33 mm (i.e., 16 French). The fluid deflecting member 534 of delivery system 53 is advantageously made of a flexible and resilient material, in particular an elastomeric material, and as such can be larger than the diameter of the catheter body. The catheter body can have a length between 65 and 85 cm.

## Claims

1. Catheter (10) for delivering an agent, comprising:
a catheter body (11) comprising a first lumen (112) extending from a proximal end (101) to a distal end (102) and defining a longitudinal axis (103),
a first outlet (113, 333) adjacent to the distal end and in fluid communication with the first lumen (112),
**characterised in that** the catheter comprises fluid deflecting means (132, 331, 431, 531) configured to deflect a flow of the agent discharged from the first outlet (113, 333) in a direction having a backwards component relative to the flow in the first lumen, the fluid deflecting means comprising a fluid deflecting surface (137, 335, 436, 536) arranged opposite the first outlet (113, 333).

2. Catheter of claim 1, wherein the fluid deflecting surface is inclined with respect to the longitudinal axis at an angle between 95° and 180° measured from a distal side of the longitudinal axis (103).

3. Catheter of claim 1 or 2, comprising a valve (14) configured to close the first lumen (112) from ambient environment.

4. Catheter of any one of the preceding claims, wherein the fluid deflecting means comprises a fluid channel (132) having a second outlet (133) arranged at a proximal side compared to the first outlet (113), wherein the fluid channel extends parallel to the first lumen between the first outlet (113) and the second outlet (133).

5. Catheter of claim 4, wherein the first outlet is arranged in a side wall of the first lumen and wherein a side wall (137) of the fluid channel (132) forms the fluid deflecting surface.

6. Catheter of claim 4 or 5, wherein the fluid deflecting means comprises a second fluid deflecting surface (135) arranged opposite the second outlet (133), wherein the second fluid deflecting surface (135) is arranged to impart a direction change of the flow of the agent discharged from the second outlet (133) compared to the flow of the agent through the fluid channel (132).

7. Catheter of claim 6, wherein the fluid deflecting means (131) comprises a radial protrusion (134) attached to the catheter body (11), the second fluid deflecting surface (135) being a surface of the radial protrusion.

8. Catheter of any one of the preceding claims, wherein the fluid deflecting means (331, 431, 531) comprises a second lumen (332) extending from the proximal end (101) to the distal end (102) adjacent to the first lumen and a fluid deflecting member (334, 434, 534), wherein the second lumen comprises a second outlet (338, 538), wherein the fluid deflecting member is configured to deflect a fluid discharged from the second outlet (338, 538) towards an outlet (113, 333) of the first lumen, thereby intersecting a fluid path exiting the first lumen.

9. Catheter of claim 8, wherein the outlet of the first lumen is the first outlet (133), wherein the fluid deflecting member is a cap (334, 434, 534) arranged opposite the second outlet (338) and spaced apart from the second outlet (338, 538).

10. Catheter of claim 9, wherein the fluid deflecting surface (335, 436, 536) extends at an edge of the cap (334, 434, 534).

11. Catheter of claims 9 or 10, wherein the cap (436, 536) is fixed to the catheter body (11).

12. Catheter of claim 11, wherein the fluid deflecting surface (436) forms a portion of an inner wall of a hole (435) through the cap (434).

13. Catheter of any one of the claims 9 to 11, wherein the cap (534) is dome-shaped and spaced apart from the first outlet (333).

14. Catheter of any one of the claims 1 to 11, wherein the fluid deflecting surface is moveable between a first position wherein the fluid deflecting surface (135, 335) abuts against an edge (142, 342) of the first outlet and a second position wherein the fluid deflecting surface is spaced apart from the first outlet.

15. Assembly for delivering an agent, comprising the catheter (10) of any one of the preceding claims, and a supply system (12) comprising an output port configured for connection to the catheter body (11) at the proximal side (101), wherein the supply system comprises a Venturi mixing device (123) configured to prepare a mixture of a fluid and the agent, wherein an output of the Venturi mixing device is fluidly coupled with the first lumen (112).
